# EUROPEAN PATENT SPECIFICATION

(11) **EP 1 848 448 B1**
(45) Date of publication and mention of the grant of the patent: **16.06.2010**
(21) Application number: 05707259.7
(22) Date of filing: 08.02.2005
(51) Int. Cl.: A61K 35/74, A61K 31/133, A61K 31/453, A61K 31/495, A61P 31/06

(54) **USE OF A BACTERIAL LYSATE FOR THE PREVENTION OF TUBERCULOSIS RELAPSE**
VERWENDUNG EINES BAKTERIENLYSATS ZUR VERHINDERUNG EINES TUBERKULOSERÜCKFALLS
UTILISATION D'UN LYSAT BACTERIEN PERMETTANT DE PREVENIR LES RECHUTES DE TUBERCULOSE

(43) Date of publication of application: 31.10.2007
(73) Proprietor: Parmeggiani, Francesco, 16136 Genova (IT)
(72) Inventor: DYACHYK, Nataliya, 79011 L'VIV Ukraine (UA)
(74) Representative: Bianchetti, Giuseppe
(86) International application number: PCT/EP2005/001256
(87) International publication number: WO 2006/084477

(56) References cited:
- BOURA P ET AL: "Effect of bacterial extracts on the immunologic profile in chronic relapsing Brucellosis patients" INTERNATIONAL JOURNAL OF IMMUNOPATHOLOGY AND PHARMACOLOGY, vol. 12, no. 2, May 1999 (1999-05), pages 103-111, XP008054556 ISSN: 0394-6320
- ROSSI S ET AL: "EFFICACY AND SAFETY OF A NEW IMMUNOSTIMULATING BACTERIAL LYSATE IN THE PROPHYLAXIS OF ACUTE LOWER RESPIRATORY TRACT INFECTIONS A RANDOMISED OPEN, CONTROLLED CLINICAL TRIAL" ARZNEIMITTEL FORSCHUNG. DRUG RESEARCH, ECV EDITIO CANTOR VERLAG, AULENDORF, DE, vol. 54, no. 1, 2004, pages 50-56, XP008054881 ISSN: 0004-4172
- TRICARICO D ET AL: "PREVENTION OF RECURRENT UPPER RESPIRATORY TRACT INFECTIONS IN A COMMUNITY OF CLOISTERED NUNS USING A NEW IMMUNOSTIMULATING BACTERIAL LYSATE A RANDOMIZED, DOUBLE-BLIND CLINICAL TRIAL" ARZNEIMITTEL FORSCHUNG. DRUG RESEARCH, ECV EDITIO CANTOR VERLAG, AULENDORF, DE, vol. 54, no. 1, 2004, pages 57-63, XP008054882 ISSN: 0004-4172
- BORIS V M: "PROFILASSI DEGLI EPISODI DI INFEZIONE DELLE VIE AEREE DURANTE LA STAGIONE INVERNALE CON UN VACCINO ANTIBATTERICO SUBLINGUALE OTTENUTO PER LISI MECCANICA: STUDIO CLINICO SU PAZIENTI AFFETTI DA PREGRESSA MALATTIA TUBERCOLARE" GIMT - GIORNALE ITALIANO DELLE MALATTIE DEL TORACE 2003 ITALY, vol. 57, no. 3, 2003, pages 210-215, XP008055993 ISSN: 1127-0810
- OLEKSIJEW A ET AL: "In vivo efficacy of ABT-255 against drug-sensitive and -resistant Mycobacterium tuberculosis strains" ANTIMICROBIAL AGENTS AND CHEMOTHERAPY 1998 UNITED STATES, vol. 42, no. 10, 1998, pages 2674-2677, XP008054856 ISSN: 0066-4804
- ROMAIN F ET AL: "IDENTIFICATION OF A MYCOBACTERIUM BOVIS BCG 45/47-KILODALTON ANTIGEN COMPLEX, AN IMMUNODOMINANT TARGET FOR ANTIBODY RESPONSE AFTER IMMUNIZATION WITH LIVING BACTERIA" INFECTION AND IMMUNITY, AMERICAN SOCIETY FOR MICROBIOLOGY. WASHINGTON, US, vol. 61, no. 2, 1 February 1993 (1993-02-01), pages 742-750, XP002001490 ISSN: 0019-9567

## Description

### Field of the invention

The present invention relates to the treatment of tuberculosis, in particular to the prevention of tuberculosis relapse.

### Background of the invention

Tuberculosis is a pulmonary infection caused by Mycobacterium tuberculosis, Micobacterium bovis or Mycobacterium africanum. The pathogenesis comprises three stages, (1) primary or initial infection, (2) latent or dormant infection and (3) rescrudescent or adult-type tuberculosis. Tuberculosis therapy comprises the administration of antimicrobial drugs associations, such as isoniazide, pirazinamide and ethambutol. The association of more chemotherapeutics is necessary to prevent development of resistance, which is the main cause of relapse.

However, in recent years there has been a constant increase of cases of primary infections and relapse; there is therefore the need for a new preventive treatment.

### Description of the invention

It has now been found that lyophilized bacterial extracts obtained by mechanical or alkaline lysis of bacteria responsible for infections of the upper and lower respiratory tract can be effectively used for the preparation of a medicament for the prevention of tuberculosis relapse, to be administered in association with antimicrobial agents.

Romain et al., Infect. Immun., vol. 61,1993,742-50 discloses that the use of bacterial extracts of Mycobacterium bovis results in a transient and marginal protection.

Bacterial extracts suitable for carrying out the invention are known and currently used in therapy for the prevention and treatment of infections of the upper and lower respiratory tract, especially in children and in the elderly (see e.g. Boris V.M., GIMT, vol. 57, 2003, 210-5). These extracts contain microbial strains of the following microorganisms: Staphylococcus aureus, Streptococcus pneumoniae (formerly named Diplococcus pneumoniae), Streptococcus pyogenes, Streptococcus viridans, Klebsiella pneumoniae, Klebsiella ozaenae, Klebsiella pneumoniae, Haemophilus influenzae serotype B, Neisseria catarrhalis. Particularly preferred is the use of a lyophilised extract obtained by mechanical or alkaline lysis of the following bacteria: Staphylococcus aureus, Streptococcus pyogenes, Streptococcus viridans, Klebsiella pneumoniae, Klebsiella ozaenae, Haemophilus influenzae serotype B, Neisseria catarrhalis and Diplococcus pneumoniae. The bacterial extract is formulated as a sublingual vaccine, in admixture with pharmaceutically acceptable excipients, for example those reported in Remington's Pharmaceutical Sciences Handbook, XVII ed. Mack Pub., N.Y., U.S.A.; particularly preferred are the preparations marketed under the name Ismigen®; in the following description, for the sake of conciseness, reference will be made to O-BAC™ ("obtrita bacteria"). According to a preferred embodiment, the vaccine is formulated as an association with isoniazide, pirazinamide and ethambutol or as a combined preparation containing a lyophilised bacterial extract as defined above, isoniazide, pirazinamide and ethambutol for simultaneous, separate, sequential administration. A further object of the present invention is a combined preparation containing a lyophilised bacterial extract as defined above, isoniazide and/or pirazinamide and/or ethambutol for simultaneous, separate, sequential administration for the prevention of tuberculosis relapse.

The vaccine will be administrated at the dose of 10 mg/day for 10 days a month for two consecutive months, in association with isoniazide and/or ethambutol and/or pirazinamide, but the dosage and the administration regimen can be adjusted by those skilled in the art of medicine according to the patient's needs.

The invention will be now illustrated in greater detail in the experimental section.

### Experimental section

### Materials and methods

Our study was carried out on 280 subjects who had contracted the infection and 30 healthy volunteers.

Inclusion criteria were age comprised between 50 and 79, negative analysis to BPCO and previous infection. Exclusion criteria were the concomitance of serious neurological and cardiovascular diseases, kidney and liver insufficiency and significant immune system alterations or previous prophylaxis therapies with bacterial lysates or other drugs interfering with the immune response in the previous 18 months.

The study was carried out over a two months period in autumn (2003), when prophylaxis is usually undertaken.

The treated group was randomly divided in two subgroups of 190 people each. The first subgroup, further to the standard antibiotic treatment against tuberculosis relapse (an association of isoniazide, ethambutol and pirazinamide), received also O-BAC™ by sublingual route, at the dose of 10 mg/day for 10 days a month for two consecutive months.

The second group of patients received the standard antibiotic treatment and a placebo by sublingual route, following the same administration regimen as the first group.

The patients of both groups were submitted to hematochemical tests in order to evaluate the immune system function (RTBL with PhHA, PPD, TIC9) before and after treatments. IgE and specific andibodies for anti-streptococcus, anti-staphylococcus and anti-pneumococcus antibodies have also been counted.

The efficacy of the treatment was also evaluated from the clinical standpoint as number of relapses and concomitant respiratory infections.

### Results

Tables 1 and 2 report the results of the clinical study described above.

The differences between the group treated with O-BAC™ and the non-treated one are statistically meaningful. In the treated group concomitant respiratory infections were one-third of those observed in the non-treated group (37 versus 122) and the number of relapses in the treated group was half the number of non-treated one (8 cases versus 36).

28.6% of the treated patients showed an increase in T-lymphocytes activity (RTBL with PhHA) from 21.7±1.3% before treatment to 34.1±15% after administration of O-BAC™. Reduction of RBTL PPD, TIC, IgE, ME and increase of antibodies against streptococcus, staphylococcus and pneumococcus was also observed.

Variations of immune function parameters in patients not receiving O-BAC™ were less relevant than in the treated group and in most cases not statistically meaningful (sometimes the variations even had the opposite sign).

These results demonstrate that lyophilised bacterial extracts in association with antibacterial therapy reduce to half the incidence of relapses and can therefore be effectively used for the prevention of tuberculosis relapse.

**Table 1 - Clinical efficacy of the treatment with BMBL**

| | Number of patients in both groups | Treatment with O-Bac^{™} | | | Treatment without O-Bac^{™} | | |
|---|---|---|---|---|---|---|---|
| | | N. of patients | sick patients | % | N. of patients | sick patients | % |
| Number of episodes of intercurrent disease | 280 | 140 | 37 | 26.4 | 140 | 112 | 80.0 |
| Number of tubercolosis relapses | 280 | 140 | 18 | 12.8 | 110 | 36 | 25.7 |

**Table 2 - Clinical Efficacy of the Treatment with O-BAC™ on the main laboratory parameters**

| Criteria | Healthy | Control group receiving placebo (n = 140) | | P | Treated group receiving O-Bac™ | | P |
|---|---|---|---|---|---|---|---|
| | | before | after | | before | after | |
| RBTL with PhHa | 32.5±2.7% | 20.0±2.6% | 26.1±1.2% | ns | 21.7±1.3% | 34.1±1.5% | <0.05 |
| Fused Protein Derivated PPD % | 1.5±0.5 | 3.3±0.3 | 2.6±0.3 | ns | 3.0±0.3 | 2.3±0.2 | <0.05 |
| Turn immunology complex TIC | 78.5±7.0 | 134.0±4ml | 128.1±6.2 | ns | 210.0±10.1 | 280.0±21.0 | <0.05 |
| Ig E MO | 175.0±15.0 | 260.0±20.1 | 230.2±10.1 | ns | 210.0±10.1 | 278.0±21.0 | <0.05 |
| Anti-pneumococcus antibodies | 4.5±0.5 | 6.5±0.3 | 6.0±0.3 | ns | 12.5±0.4 | 17.0±0.2 | <0.05 |
| Anti-streptococcus antibodies | 5.5±0.5 | 10.2±2.6 | 6.8±0.3 | <0.05 | 10.8±0.4 | 16.1±0.4 | <0.05 |
| Anti-staphylococcus antibodies | 5.5±0.5 | 8.1±0.4 | 6.0±0.3 | ns | 8.8±0.4 | 15.1±0.3 | <0.05 |

## Claims

1. Use of a bacterial lysate of Staphylococcus aureus, Streptococcus pyogenes, Streptococcus viridans, Klebsiella pneumoniae, Klebsiella ozaenae, Haemophilus influenzae serotype B, Neisseria catarrhalis and Diplococcus pneumoniae for the preparation of a medicament for the prevention of tuberculosis relapse, **to be administered in association with antimicrobial agents.**

2. The use according to claim 1 wherein the lysate is a lyophilised lysate obtained by mechanical or alkaline lysis.

3. The use according to any one of claims 1 to 2 wherein the medicament further contains isoniazide and/or pirazinamide and/or ethambutol.

4. A combined preparation containing a lyophilised bacterial extract as defined in any one of claims 1 to 3, isoniazide and/or, pirazinamide and/or ethambutol for simultaneous, separate, sequential administration for the prevention of tuberculosis relapse.

## Patentansprüche

1. Verwendung eines bakteriellen Lysats aus Staphylococcus aureus, Streptococcus pyogenes, Streptococcus viridans, Klebsiella pneumoniae, Klebsiella ozaenae, Haemophilus influenzae Serotyp B, Neisseria catarrhalis und Diplococcus pneumoniae zur Zubereitung eines Medikaments für die Verhinderung eines Tuberkuloserückfalls zur Verabreichung im Zusammenhang mit antimikrobiellen Mitteln.

2. Die Verwendung nach Anspruch 1, wobei das Lysat ein lyophilisiertes Lysat ist, das durch mechanische oder alkalische Lyse gewonnen wird.

3. Die Verwendung nach einem der Ansprüche 1 bis 2, wobei das Medikament ferner Isoniazid und/oder Pirazinamid und/oder Ethambutol enthält.

4. Ein Kombinationspräparat, das einen lyophilisierten bakteriellen Extrakt, wie er in einem der Ansprüchen 1 bis 3 definiert ist, Isoniazid und/oder Pirazinamid und/oder Ethambutol enthält, zur gleichzeitigen, getrennten, aufeinanderfolgenden Verabreichung zur Verhinderung eines Tuberkuloserückfalls.

## Revendications

1. Utilisation d'un lysat bactérien de Staphylococcus aureus, Streptococcus pyogenes, Streptococcus viridans, Klebsiella pneumoniae, Klebsiella ozaenae, Haemophilus influenzae de sérotype B, Neisseria catarrhalis et Diplococcus pneumoniae pour la préparation d'un médicament pour la prévention de la rechute de tuberculose, à administrer en association avec des agents antimicrobiens.

2. Utilisation selon la revendication 1, dans laquelle le lysat est un lysat lyophilisé obtenu par lyse mécanique ou alcaline.

3. Utilisation selon l'une quelconque des revendications 1 à 2 dans laquelle le médicament contient en outre de l'isoniazide et/ou du pirazinamide et/ou de l'éthambutol.

4. Préparation combinée contenant un extrait bactérien lyophilisé tel que défini dans l'une quelconque des revendications 1 à 3, de l'isoniazide et/ou du pirazinamide et/ou de l'éthambutol pour une administration simultanée, séparée, séquentielle pour la prévention de la rechute de tuberculose.
